# EUROPÄISCHE PATENTSCHRIFT

(11) **EP 1 799 282 B1**
(45) Veröffentlichungstag und Bekanntmachung des Hinweises auf die Patenterteilung: **19.03.2008**
(21) Anmeldenummer: 05787175.8
(22) Anmeldetag: 23.09.2005
(51) Int. Cl.: A61M 1/10

(54) **INTRAKARDIALE BLUTPUMPE**
INTRACARDIAC BLOOD PUMP
POMPE SANGUINE INTRACARDIAQUE

(30) Priorität: 14.10.2004 DE 102004049986
(43) Veröffentlichungstag der Anmeldung: 27.06.2007
(73) Patentinhaber: Abiomed Europe GmbH, 52074 Aachen (DE)
(72) Erfinder: SIESS, Thorsten, 52146 Würselen (DE); KIRCHHOFF, Frank, 52531 Übach-Palenberg (DE)
(74) Vertreter: Selting, Günther
(86) Internationale Anmeldenummer: PCT/EP2005/054767
(87) Internationale Veröffentlichungsnummer: WO 2006/040252

(56) Entgegenhaltungen:
- DE-A1- 10 040 403

## Beschreibung

Die Erfindung betrifft eine intrakardiale Blutpumpe mit einem einen Motor enthaltenen Antriebsteil, einem Pumpenteil, der ein von dem Motor angetriebenes Pumpenrad enthält, wobei zwischen dem Antriebsteil und dem Pumpenteil seitliche Ausströmöffnungen vorgesehen sind, und mit einem an dem Pumpenteil befestigten, die Ausströmöffnungen bedeckenden Schirm.

Eine intrakardiale Blutpumpe ist eine Blutpumpe, die mindestens teilweise in das Herz eingeführt wird, um Blut aus dem Herzen in eine Arterie zu fördern, wobei die Pumpe durch eine Operationsöffnung des Herzens hindurchragen kann. Solche intrakardialen Blutpumpen haben einen maximalen Außendurchmesser von etwa 10 bis 15 mm. Eine spezielle Form intrakardialer Blutpumpen sind intravasale Blutpumpen. Diese werden durch das Gefäßsystem des Patienten hindurch in das Herz eingeführt, wobei die Inzisionsstelle sich im Abstand vom Herzen befindet. Intravasale Blutpumpen haben einen Durchmesser von maximal etwa 8 mm und eine rigide Länge von maximal 35 mm. Die Förderleistung beträgt ca. 4,5 l/min bei physiologischen Drücken von (60 bis 80 mmHg).

DE 100 40 403 A1 (Impella) beschreibt eine intrakardiale Blutpumpe, von der der Oberbegriff des Patentanspruchs 1 ausgeht. Die Ausströmöffnungen sind von einem rohrförmigen Schirm bedeckt. Der Schirm bewirkt eine wirkungsmäßige Verkürzung der Baulänge der Blutpumpe, wenn die Blutpumpe in einer Herzklappe platziert wird und in eine Arterie hinein fördert. Dies erfordert es, dass der Schirm ein langgestrecktes Rohr bildet, das einen wesentlichen Teil des Pumpenteils und des Antriebsteils überdeckt.

Eine weitere intrakardiale Pumpvorrichtung, die in DE 103 36 902 B3 (Impella) beschrieben ist, weist eine Pumpe auf, die am distalen Ende mit einer flexiblen Kanüle verbunden ist. Am distalen Ende der Kanüle befindet sich ein Saugkopf zum Ansaugen des Bluts. Der Saugkopf ist mit einem nichtsaugenden Fortsatz verlängert. Dieser stabilisiert die Position der Pumpvorrichtung im Herzen.

Bei einer intrakardialen Blutpumpe ist die Erzielung einer großen Fördermenge wichtig. Die Fördermenge kann durch Erhöhung der Pumpendrehzahl vergrößert werden, jedoch sind hier wegen der Gefahr der Blutschädigung Grenzen gesetzt.

Der Erfindung liegt die Aufgabe zugrunde, eine intrakardiale Blutpumpe zu schaffen, die eine erhöhte Fördermenge liefert.

Die Blutpumpe der vorliegenden Erfindung ist durch den Patenanspruch 1 bezeichnet. Erfindungsgemäß ist vorgesehen, dass der Schirm an dem stromauf liegenden Ende der Ausströmöffnungen von dem Pumpenteil abgeht, derart, dass die die Ausströmöffnungen verlassende Strömung stoßfrei auf einen schrägen, sich in die proximale Richtung stetig erweiternden Führungsbereich des Schirmes übergeleitet wird.

Die Erfindung schafft einen stoßfreien glatten Übergang von dem Inneren der Pumpe in die axial gerichtete Außenströmung. Sie beruht auf der Erkenntnis, dass nach dem Stand der Technik beim Durchtritt des Blutes durch die Ausströmöffnungen Stoßverluste und Verwirbelungen auftreten, durch die ein großer Anteil der kinetischen Energie durch Dissipation verloren geht. Erfindungsgemäß werden diese Verluste durch die strömungsleitende Wirkung des Schirmes vermieden. Der Schirm hat somit nicht die Aufgabe des Freihaltens der Ausströmöffnungen, sondern er bewirkt das Überleiten der die Ausströmöffnungen passierenden Strömung auf einen schrägen Führungsbereich des Schirmes. Dieser schräge Führungsbereich schließt sich unmittelbar an die Ausströmöffnungen an, so dass weder Verwirbelungszonen noch Totwasserbereiche entstehen. Versuche haben gezeigt, dass durch diese Gestaltung des Schirmes die Förderleistung der Pumpe bei sonst gleichen Verhältnissen (gleiche Druckdifferenz und gleiche Drehzahl) um 10 bis 20 % gesteigert werden kann.

Gemäß einer bevorzugten Ausgestaltung der Erfindung ist der Schirm flexibel. Dies bedeutet, dass der Schirm durch Deformierung zusammengefaltet und an die Außenwand der Pumpe angelegt werden kann. Ohne äußere Krafteinwirkung nimmt der Schirm selbsttätig seine Arbeitsposition ein. Der Schirm besteht beispielsweise aus einem dünnen Häutchen aus Polyurethan mit einer Stärke von 0,1 bis 0,2 mm. Andererseits muss der Schirm eine solche Steifheit haben, dass er auch bei der durch die Strömung hervorgerufenen hydraulischen Druckabsenkung offen bleibt.

Vorzugsweise hat das Pumpenrad Flügel, die sich bis unter die Ausströmöffnungen erstrecken. Dadurch kann die Baulänge der Pumpe verringert und gleichzeitig die hydraulische Leistung erhöht werden. Die unter den Ausströmöffnungen liegenden Teile der Flügel fördern radial direkt gegen den schrägen Schirm. Der Schirm verhindert auch, dass Körpergewebe in den Bereich der Flügel gelangt und von diesen beschädigt wird. Somit trägt der Schirm dazu bei, die Produktsicherheit der intrakardialen Blutpumpe zu erhöhen.

Der Schirm kann um mehr als 90 Grad entgegen der Strömungsrichtung umgekrempelt und an dem Pumpenteil angelegt werden. Dies ist erforderlich, wenn die Blutpumpe durch eine rohrförmige Schleuse hindurch in den Körper eingeführt wurde und durch dieselbe Schleuse zurückgezogen werden soll. Während sich beim Vorschieben der Pumpe der flexible Schirm gegen die Außenwand der Pumpe anlegen lässt, würde der Schirm im aufgespannten Zustand das Zurückziehen der Pumpe durch die Schleuse verhindern. Die Möglichkeit, den Schirm umzukrempeln bzw. umzufalten, hat zur Folge, dass der Schirm im gewendeten Zustand an den Pumpenteil gedrückt wird, so dass in diesem Zustand die Pumpe durch die rohrförmige Schleuse hindurch zurückgezogen werden kann.

Der Führungsbereich des Schirmes kann kegelförmig sein oder konkav gewölbt sein, beispielsweise eine exponentielle Erweiterung bilden. In jedem Fall ist der Schirm über seine gesamte Länge stetig erweitert.

Eine spezielle Ausführungsform der Erfindung sieht vor, dass am erweiterten Ende des Schirmes Halteorgane angreifen, die am Antriebsteil befestigt sind und beim Einziehen der Blutpumpe in eine Schleuse den Schirm radial einziehen.

Der Schirm kann integraler Bestandteil einer Kanüle sein, die auf dem Pumpenteil befestigt ist. Andererseits kann auch der Schirm an dem Pumpenteil durch Kleben oder auf andere geeignete Weise befestigt sein.

Im folgenden werden Ausführungsbeispiele der Erfindung unter Bezugnahme auf die Figuren näher erläutert.

Es zeigen:
- Fig. 1: eine Ansicht der intrakardialen Blutpumpe mit für die Platzierung eingebrachtem Führungsdraht,
- Fig. 2: einen Längsschnitt durch den Pumpenteil,
- Fig. 3: den Pumpenteil von Fig. 2, mit einem hindurch gesteckten Führungsdraht, und
- Fig. 4: ein Ausführungsbeispiel, bei welchem ein Zusammenlegen des Schirmes beim Zurückziehen in eine Schleuse erfolgt.

Die intrakardiale Pumpvorrichtung weist einen Antriebsteil 10 und einen hierzu koaxialen Pumpenteil 11 auf. Der Antriebsteil 10 enthält einen (nicht dargestellten) Motor. Das proximale Ende des Antriebsteils 10 ist mit einem Katheter 12 verbunden, der die elektrischen Leitungen für den Betrieb und die Steuerung der Blutpumpe enthält. Der Pumpenteil 11 ist mit einer Kanüle 13 verbunden, die aus einem langgestreckten flexiblen Schlauch besteht, der an seinem distalen Ende einen Saugkopf 14 mit Einströmöffnungen 15 trägt. An den Saugkopf 14 schließt sich ein weich-elastischer Fortsatz 16 an, der die Kanüle 13 mechanisch verlängert, nicht aber hydraulisch. Dieser Fortsatz 16 ist mit einer Pigtale-Spitze versehen, um atraumatische Abstützungen am Körpergewebe zu ermöglichen.

Die Blutpumpe entspricht in ihrem generellen Aufbau der in DE 103 36 902 B3 beschriebenen Pumpvorrichtung.

Der Pumpenteil 11 weist einen Pumpenring 20 auf, der über längslaufende Stege 21. mit dem Antriebsteil 10 verbunden ist. Zwischen den Stegen 21 befinden sich die Ausströmöffnungen 22, durch die das Blut radial austritt, um anschließend an der Außenseite des Antriebsteils 10 entlang zu strömen.

Erfindungsgemäß ist an dem Pumpenteil 11 der Schirm 25 vorgesehen. Dieser weist eine ringförmige Manschette 26 auf, die auf dem Pumpenring 20 sitzt, und einen vom Pumpenring in die proximale Richtung abstehenden Führungsbereich 27, der sich stetig erweitert. Der Anfang des Führungsbereichs 27 befindet sich an dem stromaufliegenden Ende der Ausströmöffnungen 22, also an demjenigen Ende, das an den Pumpenring 20 angrenzt.

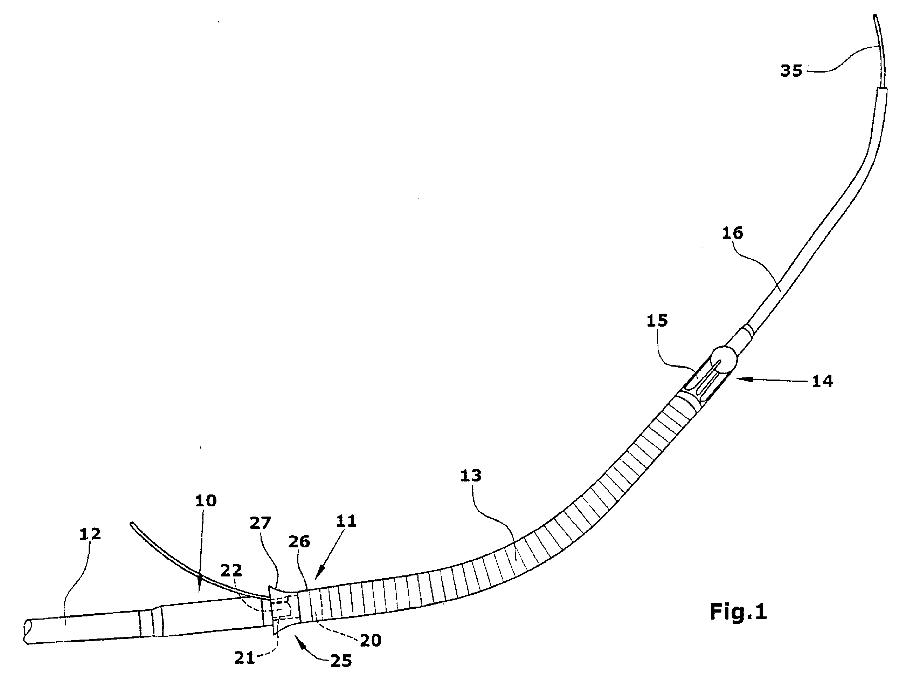

Bei dem Ausführungsbeispiel von Fig. 1 hat der Schirm 25 konkave Form. Er hat einen exponentiellen Verlauf, wobei der Führungsbereich 27 sich progressiv erweitert. Der Schirm 25 ist ringförmig und umfangsmäßig geschlossen.

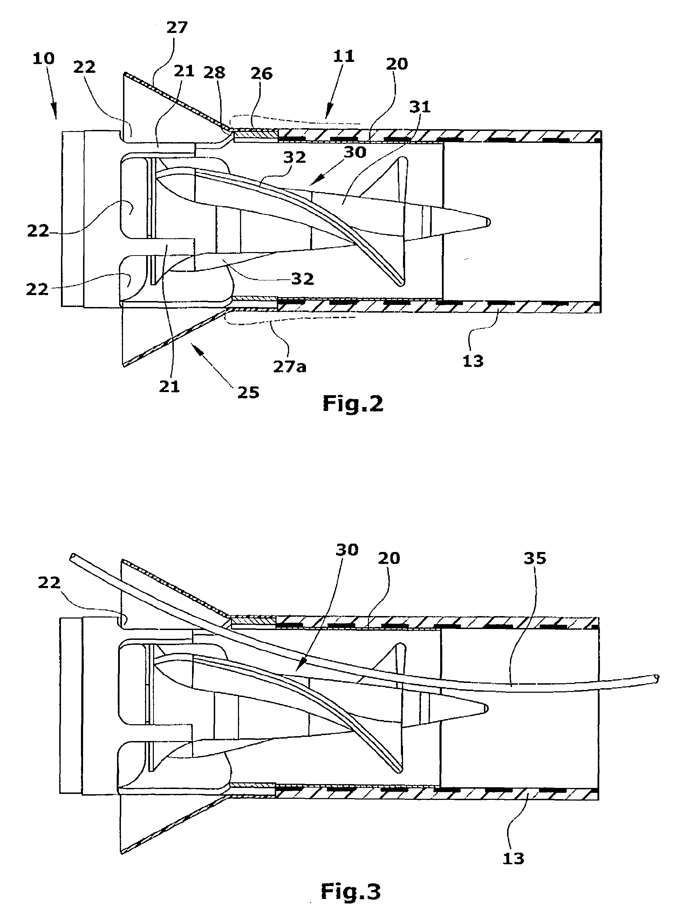

Fig. 2 zeigt einen vergrößerten Längsschnitt durch den Pumpenteil. Das Pumpenrad 30 erstreckt sich in Längsrichtung durch den Pumpenring 20. Es weist eine Nabe 31 mit radial abstehenden wendelförmigen Flügeln 32 auf. Die Nabe 31 erweitert sich im Durchmesser in Strömungsrichtung. Die Flügel 32 erstrecken sich bis in den Bereich der Ausströmöffnungen 22. Mehr als die halbe Länge der Ausströmöffnungen 22 überlagert die Flügel 32.

Der Führungsbereich 27 schließt sich unmittelbar an das stromauf liegende Ende 28 der Ausströmöffnungen 22 an. Der Führungsbereich 27 ist in Fig. 2 kegelförmig.

Die Strömung, die von dem Pumpenrad 30 in axialer Richtung gefördert wird, wird von der Nabe 31 radial nach außen abgelenkt und von dem schrägen Führungsbereich 27 des Schirmes 25 sanft wieder in axiale Richtung umgelenkt. Die Länge des Schirmes 25 ist so groß, dass der Schirm gerade die Ausströmöffnungen 22 überlagert.

Der Außendurchmesser des Antriebsteiles 10 und des Pumpenteiles 11 beträgt bei einer praktischen Ausführungsform einer intravasalen Blutpumpe 4,0 mm.

Der Außendurchmesser des Schirmes 25 beträgt 5,6 bis 6,0 mm. Die Wandstärke des Schirmes beträgt 0,1 bis 0,2 mm. Der Schirm besteht aus flexiblem Material, beispielsweise aus Polyurethan. Er kann einstückig mit der Kanüle 13 ausgebildet sein.

Fig. 3 zeigt den gleichen Pumpenteil wie Fig. 2, jedoch mit einem zusätzlich eingeführten Führungsdraht 35. Der Führungsdraht, der dazu dient, die Blutpumpe zu platzieren, wird zunächst in den Körper des Patienten eingeschoben. Dann wird die Blutpumpe mit dem Katheter darüber geschoben. Der Führungsdraht wird seitlich am Antriebsteil 10 vorbeigeführt und er tritt dann in eine der Ausströmöffnungen 22 ein, um anschließend durch die Kanüle 13 und den Fortsatz 16 zu verlaufen. Nach dem Verlegen des Katheters mit der Blutpumpe wird der Führungsdraht herausgezogen.

Die intravasale Blutpumpe wird durch eine rohrförmige Schleuse in den Patientenkörper eingeführt. Dabei ist der Außendurchmesser der Blutpumpe etwas kleiner als der Innendurchmesser der Schleuse. Während des Einführens legt sich der flexible Schirm 25 an die Außenfläche der Blutpumpe an, wobei die Ausströmöffnungen 22 verschlossen werden. Wenn die Blutpumpe aus dem distalen Ende der Schleuse ausgetreten ist, faltet sich der Schirm 25 selbsttätig auf. Ein Problem besteht darin, die Blutpumpe wieder aus dem Patientenkörper zu entfernen. Beim Zurückziehen würde sich der Schirm 25 an dem distalen Ende der Schleuse verhaken und ein Zurückziehen der Blutpumpe durch die Schleuse verhindern. Infolge der Flexibilität des Schirmes 25 legt sich der Führungsbereich des Schirmes um, so dass der Schirm um die Manschette 26 um mehr als 90 Grad umgeklappt wird und sich dann gegen die Kanüle 13 legt. In diesem Zustand kann die Blutpumpe durch die Schleuse zurückgezogen werden. Der zurückgefaltete Führungsbereich 27a ist in Fig. 2 gestrichelt dargestellt.

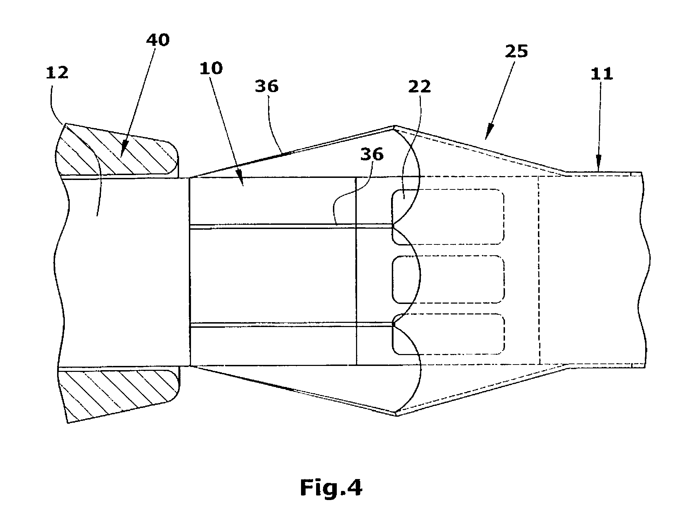

Fig. 4 zeigt ein anderes Ausführungsbeispiel. Hierbei ist das äußere Ende des Schirmes 25 mit stabförmigen Halteorganen 36 verbunden, die an dem Antriebsteil 10 befestigt sind. In Fig. 4 ist das distale Ende einer Schleuse 40 dargestellt, durch die die Blutpumpe hindurchgeschoben wird. Beim Zurückziehen stoßen die Halteorgane 36 gegen das Ende der Schleuse 40 und legen sich an die Außenfläche der Blutpumpe, wobei sie den Schirm 25 nach Art eines Segels mitnehmen.

Ein typischer Innendurchmesser der Schleuse 40 für einen Katheter von 12F (entsprechend einem Außendurchmesser von 4 mm) beträgt 4,33 mm (13F).

## Patentansprüche

1. Intrakardiale Blutpumpe mit einem einen Motor enthaltenen Antriebsteil (10), einem Pumpenteil (11), der ein von dem Motor angetriebenes Pumpenrad (30) enthält, wobei zwischen dem Antriebsteil (10) und dem Pumpenteil (11) seitliche Ausströmöffnungen (22) vorgesehen sind, und mit einem an dem Pumpenteil (11) befestigten, die Ausströmöffnungen (22) bedeckenden Schirm (25),
**dadurch gekennzeichnet,**
**dass** der Schirm (25) an dem stromaufwärts liegenden Ende (28) der Ausströmöffnungen (22) von dem Pumpenteil (11) abgeht, derart, dass die die Ausströmöffnungen (22) verlassende Strömung stoßfrei auf einen schrägen, sich in die proximale Richtung stetig erweiternden Führungsbereich (27) des Schirmes (25) übergeleitet wird.

2. Blutpumpe nach Anspruch 1, **dadurch gekennzeichnet, dass** der Schirm (25) flexibel ist.

3. Blutpumpe nach Anspruch 1 oder 2, **dadurch gekennzeichnet, dass** das Pumpenrad (30) Flügel (32) aufweist, die sich bis unter die Ausströmöffnungen (22) erstrecken.

4. Blutpumpe nach Anspruch 3, **dadurch gekennzeichnet, dass** mindestens die halbe Länge der Ausströmöffnungen (22) die Flügel (32) überlagert.

5. Blutpumpe nach Anspruch 2, **dadurch gekennzeichnet, dass** der Schirm (25) um mehr als 90 Grad entgegen der Strömungsrichtung umgelegt und an dem Pumpenteil (11) angelegt werden kann.

6. Blutpumpe nach einem der Ansprüche 1-5, **dadurch gekennzeichnet, dass** der Führungsbereich (27) des Schirmes (25) kegelförmig ist.

7. Blutpumpe nach einem der Ansprüche 1-5, **dadurch gekennzeichnet, dass** der Führungsbereich (27) des Schirmes (25) exponentiell erweitert ist.

8. Blutpumpe nach einem der Ansprüche 1-7, **dadurch gekennzeichnet, dass** der Schirm (25) über seine gesamte Länge stetig erweitert ist.

9. Blutpumpe nach einem der Ansprüche 1-8, **dadurch gekennzeichnet, dass** am erweiterten Ende des Schirms (25) Halteorgane (36) angreifen, die am Antriebsteil (10) befestigt sind und beim Einziehen der Blutpumpe in eine Schleuse (40) den Schirm (25) radial einziehen.

10. Blutpumpe nach einem der Ansprüche 1-9, **dadurch gekennzeichnet, dass** auf dem Pumpenteil (11) eine Kanüle (13) befestigt ist und der Schirm (25) integraler Bestandteil der Kanüle (13) ist.

## Claims

1. An intracardiac blood pump comprising a drive portion (10) including a motor, a pump portion (11) including a pump wheel (30) driven by said motor, wherein lateral outflow openings (22) are provided between the drive member (10) and the pump portion (11), and a shield (25) fastened to the pump portion (11) and covering the outflow openings (22),
**characterized in**
**that** said shield (25) extends from the pump portion (11) on the upstream end (28) of the outflow openings (22) in a configuration causing the outflow emerging from the outflow openings (22) to be guided in an impact-free manner onto an oblique guiding region (27) of the shield (25), said guiding region (27) continuously flaring in the proximal direction.

2. The blood pump according to claim 1, **characterized in that** the shield (25) is flexible.

3. The blood pump according to claim 1 or 2, **characterized in that** the pump wheel (30) comprises vanes (32) extending into a region below the outflow openings (22).

4. The blood pump according to claim 3, **characterized in that** at least half the length of the outflow openings (22) is superposed over the vanes (32).

5. The blood pump according to claim 2, **characterized in that** the shield (25) is adapted to be deflected by more than 90° opposite to the flow direction and to be abutted on the pump portion (11).

6. The blood pump according to any one of claims 1-5, **characterized in that** the guiding region (27) of the shield (25) is conical.

7. The blood pump according to any one of claims 1-5, **characterized in that** the guiding region (27) of the shield (25) is flared exponentially.

8. The blood pump according to any one of claims 1-7, **characterized in that** the shield (25) is flared continuously throughout its length.

9. The blood pump according to any one of claims 1-8, **characterized in that** the flared end of the shield (25) is engaged by holding members (36) fastened to the drive member (10) and operative to radially draw in the shield (25) when the blood pump is pulled into a sheath (40).

10. The blood pump according to any one of claims 1-9, **characterized in that** a cannula (13) is attached on the pump portion (11) and that the shield (25) forms an integral component of the cannula (13).

## Revendications

1. Pompe sanguine intracardiaque comportant un élément d'entraînement (10) contenant un moteur, un élément de pompe (11) qui contient un rotor (30) entraîné par le moteur, dans laquelle entre l'élément d'entraînement (10) et l'élément de pompe (11) sont prévues des ouvertures latérales d'écoulement (22), et comportant un écran (25) fixé à l'élément de pompe (11) et recouvrant les ouvertures d'écoulement (22),
**caractérisée en ce que** l'écran (25) part de l'extrémité (28) se trouvant en amont des ouvertures d'écoulement (22) de l'élément de pompe (11), de sorte que le flux quittant les ouvertures d'écoulement (22) est transféré sans à-coup sur une zone de guidage (27) oblique de l'écran (25), qui s'élargit continuellement dans la direction proximale.

2. Pompe sanguine selon la revendication 1, **caractérisée en ce que** l'écran (25) est flexible.

3. Pompe sanguine selon la revendication 1 ou 2, **caractérisée en ce que** le rotor (30) présente des ailettes (32) qui s'étendent jusque sous les ouvertures d'écoulement (22).

4. Pompe sanguine selon la revendication 3, **caractérisée en ce qu'**au moins la moitié de la longueur des ouvertures d'écoulement (22) vient recouvrir les ailettes (32).

5. Pompe sanguine selon la revendication 2, **caractérisée en ce que** l'écran (25) peut être déplacé de plus de 90 degrés à l'encontre du sens d'écoulement et peut être appliqué sur l'élément de pompe (11).

6. Pompe sanguine selon l'une des revendications 1 à 5, **caractérisée en ce que** la zone de guidage (27) de l'écran (25) a une forme conique.

7. Pompe sanguine selon l'une des revendications 1 à 5, **caractérisée en ce que** la zone de guidage (27) de l'écran (25) s'élargit de façon exponentielle.

8. Pompe sanguine selon l'une des revendications 1 à 7, **caractérisée en ce que** l'écran (25) s'élargit continuellement sur toute sa longueur.

9. Pompe sanguine selon l'une des revendications 1 à 8, **caractérisée en ce qu'**à l'extrémité élargie de l'écran (25) agissent des organes de retenue (36), qui sont fixés à l'élément d'entraînement (10) et introduisent l'écran (25) dans la direction radiale dans un canal (40) lors de l'insertion de la pompe sanguine.

10. Pompe sanguine selon l'une des revendications 1 à 9, **caractérisée en ce que** sur l'élément de pompe (11) est fixée une canule (13), et **en ce que** l'écran (25) fait partie intégrante de la canule (13).
